# EUROPÄISCHE PATENTSCHRIFT

(11) **EP 0 830 376 B1**
(45) Veröffentlichungstag und Bekanntmachung des Hinweises auf die Patenterteilung: **21.01.2004**
(21) Anmeldenummer: 96919753.2
(22) Anmeldetag: 10.05.1996
(51) Int. Cl.: C07K 14/505, A61K 38/18

(54) **VERFAHREN ZUR HERSTELLUNG VON ERYTHROPOIETIN FREI VON TIERISCHEN PROTEINEN**
PROCESS FOR PRODUCING ERYTHROPOIETIN CONTAINING NO ANIMAL PROTEINS
PROCEDE DE PRODUCTION D'ERYTROPOIETINE EXEMPTE DE PROTEINES ANIMALES

(30) Priorität: 11.05.1995 EP 95107165; 21.06.1995 DE 19522461
(43) Veröffentlichungstag der Anmeldung: 25.03.1998
(60) Teilanmeldung: 03016548.4
(73) Patentinhaber: Roche Diagnostics GmbH, 68298 Mannheim (DE)
(72) Erfinder: BURG, Josef, D-82362 Weilheim (DE); SCHNEIDER, Walter, D-82362 Weilheim (DE); WRBA, Alexander, D-82377 Penzberg (DE); FÜRST, Werner, D-82377 Penzberg (DE); SELLINGER, Karl-Heinz, D-82362 Weilheim (DE)
(86) Internationale Anmeldenummer: PCT/EP1996/001988
(87) Internationale Veröffentlichungsnummer: WO 1996/035718

(56) Entgegenhaltungen:
- EP-A- 0 267 678
- EP-A- 0 358 463
- EP-A- 0 513 738
- WO-A-85/02610
- WO-A-86/04068
- WO-A-86/07594
- JOURNAL OF BIOCHEMISTRY, Bd. 107, Nr. 3, 1990, TOKYO JP, Seiten 352-359, XP002013005 IMAI NOBUO: "Physicochemical and biological comparison of recombinant human erythropoietin with human urinary erythropoietin"
- EXPERIMENTAL HEMATOLOGY, Bd. 15, Nr. 2, 1987, LAWRENCE, Seiten 171-176, XP002013006 CHOPPIN, J. ET AL: "Biochemical analyses of murine erythropoietin from plasma and from cloned erythroleukemia cells"
- NATURE, Bd. 313, 1985, LONDON GB, Seiten 806-810, XP002013007 JACOBS, K. ET AL: "Isolation and characterization of genomic and cDNA clones of human erythropoietin"
- BUNDESVERBAND DER PHARMAZEUTISCHEN INDUSTRIE BV.: "Rote Liste 1993" 1993 , EDITIO CANTOR , AULENDORF/WÜRTT XP002013009 *08070 "Erypo 2000/4000/10000" und 08071 "Recormon 1000/2000/5000"*
- BLOOD, Bd. 67, Nr. 1, 1986, NEW YORK, Seiten 71-79, XP002013008 KRYSTAL G. ET AL: "Purification of human erythropoietin to homogeneity by a rapid five step procedure"
- MURRAY P. DEUTSCHER: "Methods in enzymology, Vol 182, Guide to protein purification" 1990 , ACADEMIC PRESS INC , NEW YORK XP002013010 siehe Seite 339 - Seite 343 siehe Seite 409 - Seite 417

## Beschreibung

Gegenstand der Erfindung ist ein Verfahren zur Herstellung von Erythropoietin, welches frei von tierischen Fremdproteinen, mit Ausnahme von Proteinen der Wirtszelle, ist.

Erythropoietin(EPO) ist ein humanes Glycoprotein, welches die Bildung von Erythrozyten stimuliert. Seine Wirkung und therapeutische Anwendung ist beispielsweise in EP-B 0 148 605, Huang, S.L., Proc. Natl. Acad. Sci. USA (1984) 2708-2712, EP-B 0 205 564, EP-B 0 209 539 und EP-B 0 411 678 sowie Lai, P.H., et al., J. Biol. Chem. 261 (1986) 3116-3121 und Sasaki, H., et al., J. Biol. Chem. 262 (1987) 12059-12076 ausführlich beschrieben. Therapeutisch verwendetes Erythropoietin wird rekombinant hergestellt (EP-B 0 148 605 und EP-B 0 209 539).

Die rekombinante Herstellung von Erythropoietin erfolgt üblicherweise in CHO-Zellen, unter Zusatz von fötalem Kälberserum und gegebenenfalls Rinderinsulin im Kulturmedium. Damit enthält eine so hergestellte EPO-Präparation auch nach der Reinigung zumindest Spuren von Substanzen, die aus diesen Zusätzen stammen. Dies können beispielsweise bovine Viren und vergleichbare Agentien, Restmengen an bovinen Proteinen und/oder boviner DNA sein.

Es ist bekannt, eine serumfreie Fermentation von rekombinanten CHO-Zellen, welche ein EPO-Gen enthalten, mit den Methoden des Standes der Technik durchzuführen. Derartige Verfahren sind beispielsweise beschrieben in der EP-A 0 513 738, EP-A 0 267 678 und in allgemeiner Form von Kawamoto, T., et al., Analytical Biochem. 130 (1983) 445-453, EP-A 0 248 656, Kowar, J. und Franek, F., Methods in Enzymology 421 (1986) 277-292, Bavister, B., Expcology 271 (1981) 45-51, EP-A0481791, EP-A 0 307 247, EP-A 0 343 635, WO 88/00967. Es hat sich gezeigt, daß bei serumfreier Kultur von EPO produzierenden eukaryontischen Wirtszellen der Anteil von Proteinen der Wirtszelle an der Gesamtproteinmenge mehr als doppelt so groß ist wie bei Kultur in serumhaltigen Medien. Ebenso enthält eine solche Protein-Präparation Nukleinsäuren aus den Wirtszellen in nicht vernachlässigbarer Menge.

In der EP-A 0 267 678 wird zur Aufreinigung des in serumfreier Kultur hergestellten EPO nach Dialyse eine Ionentauscherchromatographie an S-Sepharose, eine präparative Reverse Phase-HPLC an einer C₈-Säule und eine Gelfiltrationschromatographie beschrieben. Dabei kann der Gelfiltrationschromatographieschritt durch eine Ionentauscherchromatographie an S-Sepharose fast flow ersetzt werden. Ebenso wird vorgeschlagen, vor der Ionenaustauscherchromatographie eine Farbstoffchromatographie an einer Blue Trisacryl-Säule durchzuführen.

Auf solche Weise gereinigtes EPO hat eine Reinheit von etwa 99 % und enthält jedoch damit immer noch erhebliche Mengen an Protein und DNA aus der Wirtszelle.

In der EP-A 0 513 738 ist ein Verfahren zur Herstellung von EPO in Säugerzellen beschrieben, ohne daß näher auf die Aufreinigung eingegangen wird.

Von Nobuo, I., et al., J. Biochem, 107 (1990) 352-359 ist ein Verfahren zur Reinigung von rekombinantem EPO beschrieben. Bei diesem Verfahren wird EPO jedoch vor den Reinigungsschritten mit einer Lösung von Tween® 20, Phenylmethylsulfonylfluorid, Ethylmaleinimid, Pepstatin A, Kupfersulfat und Oxamsäure behandelt. Diese Zusätze, auch wenn sie im pharmazeutischen Präparat nur noch in Spuren vorhanden sind, sind aus therapeutischer Sicht bedenklich.

Für eine therapeutische Anwendung ist es außerdem bevorzugt, wenn ein therapeutisch wirksames Präparat vollständig frei von Proteinen und Nukleinsäuren aus Säugerzellen und weitgehend frei von Proteinen und Nukleinsäuren aus der Wirtszelle ist.

Gegenstand der Erfindung ist ein Verfahren zur Herstellung eines Proteinpräparats mit Erythropoietinaktivität, welches gekennzeichnet ist durch
a) einen Gehalt von Proteinen, die aus der Wirtszelle stammen und keine biologische Funktion von Erythropoietin besitzen, von ≤ 100 ppm
b) einen Gehalt von DNA aus der Wirtszelle von ≤ 10 pg/83 µg Erythropoietin
und dadurch, daß
c) das Präparat vollständig frei von natürlichen Säugerproteinen ist, die nicht aus der Wirtszelle stammen,
durch Expression einer für das Protein codierenden DNA in einer eukaryontischen Wirtszelle, Kultivierung der Wirtszelle in einem Medium, frei von natürlichen Säugerproteinen und chromatographischer Reinigung des Proteins aus dem Zellüberstand, dadurch gekennzeichnet, daß die chromatographische Reinigung im ersten Schritt als Farbstoffaffinitätschromatographie, im zweiten Schritt als hydrophobe Chromatographie, im weiteren Schritt als Chromatographie an Hydroxyapatit, im weiteren Schritt als reversed phase Chromatographie an einem hydrophoben Träger und als nächstem Schritt als Anionenaustauschchromatographie erfolgt.
erfindungsgemäß hergestellte

Eine EPO-Präparation, die zudem frei ist von Phenylmethylsulfonylfluorid, Pepstatin A und/oder Cu-Ionen, ist bisher nicht bekannt und auch durch die Methoden des Standes der Technik, insbesondere in therapeutisch wirksamen Mengen, nicht herstellbar. Um eine homogene EPO-Präparation dieser Spezifikation, vorzugsweise in hoher Ausbeute, zu erhalten und zu isolieren, ist die Kombination der Verfahrensschritte gemäß der Erfindung erforderlich.

Unter einem Protein mit Erythropoietinaktivitiät wird ein Protein verstanden, welches die biologische Funktion von EPO besitzt. Diese biologische Funktion besteht darin, in erythroiden Vorläuferzellen Differenzierungs- und Teilungsvorgänge zu stimulieren und dadurch Erythrozyten bereitzustellen. Vorzugsweise ist dieses Protein in seinen Eigenschaften identisch oder im wesentlichen identisch mit humanem Erythropoietin und besteht aus 166 Aminosäuren, bei einem Molekulargewicht von ca. 34 - 38 kD, wobei der Anteil der Glycosylreste im Molekulargewicht ca. 40 % beträgt. Derivate und Fragmente von EPO, welche eine analoge Aktivität haben und nach Kultivierung einer EPO produzierenden Wirtszelle hergestellt werden, können ebenfalls nach den erfindungsgemäßen Verfahren in reiner Form hergestellt werden. Die DNA- und Proteinsequenz von humanem EPO ist beispielsweise in der EP-B 0 205 564 und EP-B 0 209 539 beschrieben.

Unter "vollständig frei von natürlichen Säugerproteinen" ist zu verstehen, daß im Präparat dadurch, daß bei der Kultur der Wirtszelle keine Fremdproteine aus natürlichen Quellen, wie Rinderserumalbumin oder fötales Kälberserum, zugesetzt werden, auch keine derartigen Fremdproteine in nachweisbarem Umfang enthalten sind. Das Präparat ist also völlig frei von planmäßig zugesetzten derartigen Säugerproteinen, die nicht aus der Wirtszelle stammen und sonst üblicherweise bei einer serumfreien Kultur dem Kulturmedium zur Aufrechterhaltung und Verbesserung des Zellwachstums sowie zur Optimierung der Ausbeute zugesetzt werden. Unter natürlichen Säugerproteinen werden Säugerproteine aus natürlichen Quellen, wie aus Humanmaterial oder aus tierischem Material, verstanden, nicht jedoch rekombinante Säugerproteine, die beispielsweise in Prokaryonten, wie E.coli, hergestellt wurden.

Derartige, bei der Zellkultivierung zugesetzten Säugerproteine sind beispielsweise Rinderserumalbumin, fötales Kälberserum, Transferrin (human oder Rind), Insulin (Schwein oder Rind) oder Gelatine.

Unter einer "Wirtszelle" ist eine tierische oder humane Zelle zu verstehen, deren Genom ein aktives EPO-Gen enthält, wobei dieses EPO-Gen bei Kultur der Zelle in einem serumfreien Medium transkribiert und translatiert wird. Das EPO-Gen kann in diese Wirtszelle als exogenes Gen, vorzugsweise mit Regulationselementen, eingebracht werden (vgl. z. B. EP-B 0 148 605, EP-B 0 209 539), als aktives endogenes Gen in der Wirtszelle bereits vorhanden sein oder als endogenes nicht-aktives Gen aktiviert worden sein. Eine solche Aktivierung von endogenen Genen kann beispielsweise durch gezielte Einbringung von Regulationselementen in das Genom, beispielsweise durch homologe Rekombination, erfolgen. Derartige Verfahren sind bekannt und beispielsweise in der WO 91/09955 beschrieben.

Als Wirtszellen werden üblicherweise Säugerzellen verwendet, Falls ein exogenes humanes EPO-Gen eingebracht wird, können beispielsweise als Wirtszellen CHO- oder BHK-Zellen verwendet werden. Falls zur Expression ein endogenes EPO-Gen verwendet wird, werden zweckmäßig humane Zellen, wie beispielsweise humane Nieren-, Leber- oder Lymphzellen verwendet.

Unter "Proteine, die aus der Wirtszelle stammen" sind Proteine zu verstehen, die bei der Kultivierung der das aktive EPO-Gen enthaltenden Wirtszellen entstehen und nicht die oben angeführte Spezifikation von EPO besitzen. Die Angabe des Proteinsgehalts erfolgt in ppm, bezogen auf Gewicht (w/w) . Der Gehalt dieser Proteine kann beispielsweise bestimmt werden durch einen ELISA, der auf polyklonalen Antikörpern basiert, die gegen die Proteine der Wirtszelle gerichtet sind. Derartige polyklonale Antikörper werden erhalten, indem Tiere, vorzugsweise Schafe, immunisiert werden mit einem Extrakt der Proteine der Wirtszelle (extrazelluläre und intrazelluläre Proteine). Der Test erfolgt vorzugsweise als Sandwichtest mit einem immobilisierten polyklonalen Antikörper und einem Peroxidase-markierten zweiten Antikörper. Als Standard wird der Gesamtproteinextrakt verwendet. Die untere Nachweisgrenze eines solchen Tests liegt bei etwa 15 ng Protein pro ml. Dies entspricht bei einer üblichen EPO-Konzentration von etwa 3,0 mg/ml einer Menge von 5 ppm Proteine aus der Wirtszelle als unterste nachweisbare Menge. Vorzugsweise enthält damit das erfindungsgemäße Präparat Proteine aus der Wirtszelle in einem Gehalt von 5 ppm bis 100 ppm. Besonders bevorzugt wird eine EPO-Präparation verwendet, bei der auf diese Weise keine Proteine aus der Wirtszelle mehr nachweisbar sind.

Unter "DNA aus der Wirtszelle" ist die Gesamtmenge von DNA dieser Wirtszelle (genomische, ribosomale, mitochondriale etc. DNA) zu verstehen. Dieser Gehalt schließt auch die DNA, welche für EPO codiert und beispielsweise durch Transformation mit einer exogenen DNA erhalten wurde, ein. Er wird zweckmäßig bezogen auf eine üblicherweise verwendete therapeutische Dosis von EPO von 83 µg. Die Bestimmung des Gehalts an DNA der Wirtszelle erfolgt durch einen Hybridisierungstest mit radioaktiver oder Fluoreszenzdedektion. Als Sonden-DNA wird die Gesamt-DNA aus der Wirtszelle verwendet. Diese Gesamt-DNA wird als Standard in diesem Test verwendet. Die untere Nachweisgrenze eines solchen Hybridisierungstests liegt bei etwa 1 pg DNA/83 µg EPO. Vorzugsweise enthält damit das erfindungsgemäße Präparat DNA aus der Wirtszelle in einem Gehalt von 1 - 10 pg oder besonders bevorzugt weniger als 5 pg pro 83 µg EPO. Ganz besonders bevorzugt ist in dem Präparat auf diese Weise keine DNA mehr nachweisbar (≤ 1 pg DNA).

Überraschenderweise hat sich gezeigt, daß mit dem erfindungsgemäßen Verfahren EPO vorzugsweise aus serumfreier Kultur in hoher Reinheit mit einer hohen Ausbeute erhalten werden kann. So wird gegenüber dem Verfahren, welches bei Nobuo, I., et al., J. Biochem. 107 (1990) 352-359 beschrieben ist, in einem kompetitiven ELISA-Test auf EPO eine um ca. den Faktor 2 gesteigerte Ausbeute (25% gegenüber ca. 13% bei Nobuo et al.) erhalten.

Der zum EPO-Nachweis verwendete kompetitive ELISA-Test wurde mit den Schritten: Beschichtung einer Mikrotiterplatte mit einem polyklonalen Antikörper, welcher gegen Maus Fcy gerichtet ist; Reaktion mit einem monoklonalen Antikörper gegen EPO aus Maus; Kompetition zwischen EPO-Probe und Peroxidase-markiertem EPO; Substratreaktion mit ABTS® (2,2'-Acino-di-e(3-)ethylbenzthiazolinsulfonat(6)] durchgeführt.

Vorzugsweise wird das Proteinpräparat in Chargen von 0,1 - 10 g hergestellt. Es hat sich überraschenderweise gezeigt, daß eine für therapeutische Einsatzzwecke ausreichende Reinigung von EPO nach Kultur in einem Medium, welches frei von natürlichen Säugerproteinen ist, nur dann erreicht werden kann, wenn nach der Affinitätschromatographie an einem Farbstoff als zweiter Schritt eine hydrophobe Chromatographie erfolgt. Ein Zusatz von Proteasehemmern (z.B. CuSO₄) vor der chromatographischen Reinigung wie von Nobuo, I., et al., J. Biochem. 107 (1990) 352-359 oder in der WO 86/07494 beschrieben ist überraschenderweise nicht erforderlich. Vorzugsweise erfolgt die hydrophobe Chromatographie an einem alkylierten (C₄-C₁₈) oder arylierten (vorzugsweise phenylierten oder benzylierten) Träger. Besonders bevorzugt wird ein butylierter Träger verwendet. In diesem Fall sind Ausbeute des Reinigungsverfahrens und Reinheit des Proteins besonders hoch.

Die Expression einer für EPO-codierenden DNA in einer eukaryontischen Wirtszelle kann beispielsweise durch Transfektion einer geeigneten Wirtszelle, vorzugsweise CHO-Zellen, mit einer exogenen DNA, welche für EPO codiert, durchgeführt werden. Ebenso möglich ist es, eine in der Zelle (beispielsweise humane Nierenzellen) inaktives, endogenes EPO-Gen zu aktivieren, beispielsweise durch ein Verfahren der homologen Rekombination (WO 91/09955 und WO 93/09222). Die Kultivierung der ein aktives EPO-Gen enthaltenden Wirtszellen erfolgt in einer dem Fachmann bekannten Weise in einer Kultur, welcher keine Säugerproteine aus natürlichen Quellen zugesetzt werden. Bei dieser Kultur werden jedoch üblicherweise rekombinant hergestellte (vorzugsweise in Prokaryonten , wie E.coli) Insuline, Albumine und Transferrine zugesetzt.

Ein serumfreies Medium, welches im Rahmen der Erfindung geeignet ist, enthält beispielsweise als Medium DMEM/F12 (z. B. GRH Biosciences/Hazleton Biologics, Denver, US, Best.Nr. 57 - 736), sowie zusätzlich Natriumhydrogencarbonat, L+Glutamin, D+Glucose, rekombinantes Insulin, Natriumselenit, Diaminobutan, Hydrocortison, Eisen(II)Sulfat, Aspargin, Asparginsäure, Serin und einen Stabilisator für Säugerzellen, wie z.B. Polyvinylalkohol, Methylcellulose, Polydextran, Polyethylenglycol, Pluronic F68, Plasmaexpander Polygelin (HEMACCEL) oder Polyvinylpyrolithion.

Ein wesentlicher Vorteil des erfindungsgemäßen Verfahrens zur Reinigung von EPO ist, daß es mit diesem Verfahren gelingt, EPO, welches nach serumfreier Kulturführung einen der obengenannten Stabilisatoren enthält, in hoher Ausbeute zu reinigen und den oder die Stabilisatoren soweit abzureichem, daß sie nicht mehr nachweisbar sind.

Zur Herstellung von EPO wird die das EPO-Gen enthaltende Wirtszelle durch Passagierung in kleinvolumigen Kulturen an das Medium, das keine Säugerproteine aus natürlichen Quellen enthält, adaptiert. Die adaptierten Zellen werden ggf. kryokonserviert, nach Bedarf der Zellbank entnommen und in serumfreies Medium expandiert.

Zur Aufreinigung wird vorzugsweise der zellfreie Kulturüberstand der Wirtszelle gewonnen und nach Filtration dem erfindungsgemäßen Reinigungsverfahren unterworfen.

Vor Durchführung des Reinigungsverfahrens kann, falls erforderlich, noch eine Filtration zur Abtrennung von Trübungen und/oder eine Konzentrierung durchgeführt werden.

Mit der Farbstoffchromatographie werden im ersten Schritt im wesentlichen Kontaminationen durch Proteasen beseitigt. Vorzugsweise wird als Farbstoff ein blauer Triazinfarbstoff, wie Cibachron® blau, verwendet. Ebenso geeignet sind andere Triazin-Farbstoffe. Das Trägermaterial für die Farbstoffchromatographie ist an sich unkritisch, vorzugsweise wird jedoch ein Trägermaterial auf Polysaccharidbasis, wie z. B. Sepharose, vorzugsweise Sepharose 6 fast flow, verwendet. Die Äquilibrierung der Säule erfolgt mit Puffer, pH 4,5 - 5,5, vorzugsweise 4,8 - 5,2, vorzugsweise mit Acetatpuffer oder Essigsäure. Vorzugsweise wird bei Temperaturen von 1-10°C, besonders bevorzugt bei ca. 5°C gearbeitet.

Die Elution kann durch Erhöhung der Salzkonzentration bei saurem oder neutralem pH-Wert (vorzugsweise pH 5 - 7) erfolgen. Bei basischem pH-Wert, vorzugsweise pH 8,5 - 9,5, besonders bevorzugt bei pH 8,8 - 9,2 kann die Elution auch ohne wesentliche Änderung der Salzkonzentration erfolgen.

Wesentlich für die Qualität der Reinigung ist es, daß im zweiten Schritt eine Chromatographie an einem hydrophobisierten Träger durchgeführt wird. Geeignete Adsorbermaterialien für die hydrophobe Chromatographie sind beispielsweise in Protein Purification Methods, A practical approach, Ed. Harris, E.L.V. and Angal S., IRL Press, Oxford, England (1989), S. 224 und Protein Purification, Ed. Janson, J.C., Ryden L, VCH-Verlag, Weinheim, Deutschland (1989), S. 207-226 beschrieben. Das Trägermaterial selbst ist unkritisch und kann beispielsweise Sepharose, ein Copolymerisat aus Acrylsäure und Methacrylsäure oder Kieselgel sein. Wesentlich ist, daß an diesen Träger kovalent hydrophobe Gruppen, vorzugsweise Butylgruppen, gebunden sind. Geeignete Träger sind kommerziell erhältlich (z. B. Butyl-Toyopearl von Toso Haas, Deutschland oder Butylsepharose von Pharmacia, Deutschland).

Besonders bevorzugt wird ein butylierter Träger verwendet. Andere alkylierte oder arylierte Träger binden EPO entweder teilweise irreversibel oder führen zu einer schlechteren Auftrennung.

Die Elution in der hydrophoben Chromatographie erfolgt vorzugsweise durch Erniedrigung der Salzkonzentration (z. B. mit einem Gradienten von 4 mol/1 bis 0 mol/l oder durch Zusatz von chaotropen Agentien, wie Jodid, Perchlorat oder Rhodanid oder durch Zusatz von Alkoholen, wie Glycerin, Ethylenglykol oder Isopropanol.

Die Durchführung der hydrophoben Chromatographie erfolgt besonders bevorzugt bei neutralem pH-Wert und in Gegenwart von Salz, vorzugsweise NaCl, ca. 0,75 mol/l. Es ist ebenfalls besonders bevorzugt, die hydrophobe Chromatographie, vorzugsweise in Gegenwart eines niedermolekularen Alkohols, besonders bevorzugt in Gegenwart von Isopropanol durchzufiihren. Die Konzentration des Alkohols im Elutionspuffer ist vorzugsweise etwa doppelt bis dreimal so hoch wie im Äquilibrierungspuffer, im Waschpuffer etwa doppelt so hoch wie im Äquilibrierungspuffer. Zur Äquilibrierung (Beladung des Chromatographiematerials) werden bevorzugt etwa 10 - 15 %, vorzugsweise etwa 10 % Isopropanol, bei der Elution etwa 25 % bis 35 %, vorzugsweise etwa 27 % Isopropanol und im Waschpuffer 19 % Isopropanol zugesetzt (Konzentrationsangaben auch für andere Alkohole geeignet, Angabe in Volumen%, v/v).

Die hydrophobe Chromatographie kann in einem weiten Temperaturbereich von ca. 10 - 40°C durchgeführt werden. Vorzugsweise wird jedoch bei kontrollierter Temperatur und 27 ± 2°C gearbeitet. Temperaturen unter 10°C sind wenig geeignet.

Als weiterer Reinigungsschritt, im erfindungsgemäßen Verfahren, wird eine Trennung an Hydroxyapatit durchgeführt. Zweckmäßig besteht das Säulenmaterial aus Hydroxyapatit, welcher in ein Agarosegerüst eingelagert wird. EPO bindet an diese Matrix und wird vorzugsweise bei niedrigen Phosphatkonzentrationen eluiert. Geeignetes Säulenmaterial ist beispielsweise Hydroxyapatit-Ultrogel (Biosepra, Deutschland) oder HA-Biogel HT (Biorad, Deutschland).

Die Durchführung der Chromatographie erfolgt zweckmäßig bei etwa neutralem pH-Wert. Der Elutionspuffer enthält Phosphat, vorzugsweise Kaliumphosphat, in einer Konzentration von 1 mmol/l bis 100 mmol/l, vorzugsweise ca. 10 mmol/l.

Als weiterer Schritt zur Reinigung schließt sich eine reversed phase Chromatographie an einem hydrophobisierten Träger an. Dies ist vorzugsweise der Träger, der auch für die hydrophobe Chromatographie verwendet wird. Als Chromatographiematerialien für die reversed phase Chromatographie sind beispielsweise geeignet: Phenylsepharose und Octylsepharose (Pharmacia, Schweden), Butyl -Toyopearl (Toso Haas, Deutschland) oder Propyl-TSK (Merck, Deutschland). In diesem Verfahrensschritt ist es jedoch auch bevorzugt, Träger, die längere Alkylgruppen (z. B. C₈ oder C₁₈) enthalten, zu verwenden. Die Äquilibrierung der Säule erfolgt vorzugsweise im pH-Bereich zwischen 2 und 7, vorzugsweise pH 2,5, wobei vorzugsweise wässrige Trifluoressigsäure verwendet wird. Zur Elution wird ein Gradient vom Äquilibrierungspuffer zu einer wässrigen Lösung eines polaren organischen Lösungsmittels, wie beispielsweise Acetonitril, verwendet. Nach der Chromatographie wird das Eluat zweckmäßig neutralisiert.

Als nächster Schritt des erfindungsgemäßen Reinigungsverfahrens schließt sich eine Anionenaustauschchromatographie an. Als Säulenmaterial wird hier vorzugsweise DEAE-Sepharose fast flow verwendet. Die Äquilibrierung erfolgt bei pH 6 - 9, vorzugsweise bei pH 7,5. Gegebenenfalls nach Waschen, vorzugsweise mit einer sauren Lösung (ca. pH 4,5), wird im Neutralen oder leicht Basischen (pH 6 - 9, vorzugsweise um pH 7,5, unter Erhöhung der Ionenstärke, vorzugsweise mit NaCl) eluiert. Als Puffer werden vorzugsweise Phosphatpuffer verwendet.

### Das erfindungsgemäße Verfahren wird durch die folgenden Beispiele näher erläutert:

### Beispiel

### 1. Ausgangsmaterial

EPO wird in CHO-Zellen nach dem batch-Verfahren fermentiert. Der Fermenter wird mit einer Vorkultur angeimpft und nach ca. 5 Tagen der Fermenterinhalt geerntet. Nach der Ernte werden die CHO-Zellen aus der Fermentationsbrühe durch Abzentrifugieren entfernt. Der zellfreie Kulturüberstand wird mit 1 mol/l Essigsäure auf pH 5,0 - 5, 2 eingestellt und bei 1-9°C filtriert.

Als Kulturmedium wird ein serumfreies Medium verwendet, welches besteht aus dem Basismedium DME(HG) HAM's F-12 modified (R5) (GRH Biosciences/Hazleton Biologics, Denver, USA, Best.Nr. 57 - 736), Natriumhydrogencarbonat, L-(+) Glutamin, D-(+)Glucose, rekombinantem Insulin, Natriumselenit, Diaminobutan, Hydrocortison, EisenII-Sulfat, Aspargin, Asparginsäure, Serin und Polyvinylalkohol.

### 2. Blaue-Sepharose-Chromatographie

### 2.1 Trennprinzip

Blaue Sepharose (Pharmacia) besteht aus Sepharosekügelchen, an deren Oberfläche der Farbstoff Cibacron®blau kovalent gebunden ist. EPO bindet bei niedriger Ionenstärke und neutralem bis saurem pH-Werten an diesen Träger. EPO wird durch Erhöhung der Ionenstärke und des pH-Wertes eluiert.

### 2.2 Durchführung

Die Chromatographiesäule (Amicon P440 x 500, Amicon, GB) wird mit 60 - 80 l Blaue Sepharose gefüllt und mit 0,5 N NaOH regeneriert. Anschließend wird die Säule mit ca. 3 Säulenvolumina (SV) Acetatpuffer äquilibriert. Der zellfreie, auf pH 5 eingestellt Kuturüberstand wird bei einer Temperatur von 10 ± 5 °C und einer Flußrate von 800 - 1400 ml/min auf die Säule aufgezogen. Die Säule wird bei gleicher Flußrate und 5 ± 4 °C mit ca. 1 SV Waschpuffer 1 nachgewaschen. Dann folgen ca. 2 SV Waschpuffer 2. Anschließend wird die Säule mit ca. 3 SV Elutionspuffer eluiert. Der gesamte Proteinpeak wird gesammelt (ca. 30 - 60 l), mit HCl auf pH 6,9 eingestellt und bis zur Weiterverarbeitung bei 5 ± 4 °C gelagert. Bei diesem Chromatograhieschritt wird die Produktlösung konzentriert und eine Reinheit von ca. 40 - 50 % erreicht.

| | |
|---|---|
| Äquilibrierpuffer | 20 mM Na-Acetat, 5 mM CaCl₂, 0,1 M NaCl, pH 5,0 ± 0,2 |
| Waschpuffer 1 | 20 mM Na-Acetat, 5 mM CaCl₂, 0,25 M NaCl, pH 5,0 ± 0,2 |
| Waschpuffer 2 | 20 mM Tris HCl, 5 mM CaCl₂, pH 6,5 ± 0,3 |
| Elutionspuffer | 100 mM Tris HCl, 5 mM CaCl₂, 1 M NaCl, pH 9,0 ± 0,2. |

### 3. Butyl-Toyopearl-Chromatographie (hydrophobe Chromatographie)

### 3.1 Trennprinzip

Butyl-Toyopearl (TosoHaas) ist ein Träger, an dessen Oberfläche Butyl-Reste kovalent gebunden sind. EPO bindet an diese Matrix und wird mit einem Isopropanol-haltigen Puffer eluiert.

### 3.2 Beladungs- und Elutionsbedingungen

Nach Bindung des Proteins an die Butyl-Matrix in einem Äquilibrierungspuffer, der 10 % Isopropanol enthielt, wurde das EPO durch einen Gradienten - bestehend aus wässriger Pufferlösung und 50 %igem Isopropanol- eluiert. Diese Elution beginnt ab ca. 20 % Isopropanol.

Es war zu erwarten, daß der Zusatz des Elutionsmittels Isopropanol im Äquilibrierungspuffer die Bindung von "Fremdprotein" minimiert und die Bindung von EPO schwächt (geringere Kapazität). Überraschenderweise wurde gefunden, daß der Zusatz von Isopropanol im Äquilibrierungspuffer in definierten Konzentrationen (10-15 % ), die Bindung von EPO erhöht und dabei auch die Ausbeute verbessert (vergl. Tabelle 1).

EPO bindet bei +4°C nicht an Butyl-Toyopearl. Bei +25°C werden 800µg EPO/ml Adsorber gebunden (durch Isopropanol auf 1.000 µg/ml gesteigert) und bei +35°C überraschenderweise sogar 1.700 µg EPO/ml Butyl-Toyopearl.

**Tabelle 1:**

| **EPO-Ad- und Desorption in Abhängigkeit vom Isopropanol-Zusatz** | | | | | |
|---|---|---|---|---|---|
| % Isopropanol im Äquilibrierungspuffer | 0 | 10 | 15 | 17 | 19 |
| % EPO im Waschpuffer | 24 | <1 | <1 | <1 | 10 |
| % EPO im Eluat | 76 | 96 | 93 | 83 | 69 |

### 3.3 Durchführung

Die Chromatographiesäule (Pharmacia BPG 300/500) wird mit 30 - 40 1 Butyl-Toyopearl gefüllt und mit 4 M Guanidin-HCl und 0,5 N NaOH regeneriert. Anschließend wird die Säule mit mindestens 3 SV Äquilibrierpuffer äquilibriert.

Das Eluat der Blauen Sepharose-Säule wird auf 10 % Isopropanol eingestellt und bei einer Temperatur von 27 ± 2 °C und einer Flußrate von 800 - 1200 ml/min auf die Säule aufgezogen. Die Säule wird bei gleicher Temperatur und Flußrate mit ca. 1 SV Äquilibrierpuffer und dann mit ca. 2 SV Waschpuffer nachgewaschen. Anschließend wird sie mit ca. 3 SV Elutionspuffer eluiert. Der gesamte Proteinpeak wird gesammelt (ca. 10 - 18 1), sofort mit Verdünnungspuffer um den Faktor 3 verdünnt und bis zur Weiterverarbeitung bei 15°C gelagert. Bei dieser Chromatographie wird eine Reinheit von ca. 90 % erreicht.

| | |
|---|---|
| Äquilibrierpuffer | 20 mM Tris-HCl, 5 mM CaCl₂, 0,75 M NaCl, 10 % Isopropanol, pH 6,9 ± 0,2 |
| Waschpuffer | 20 mM Tris-HCl, 5 mM CaCl₂, 0,75 M NaCl, 19 % Isopropanol, pH 6,9 ± 0,2 |
| Elutionspuffer | 20 mM Tris-HCl, 5 mM CaCl₂, 0,75 M NaCl, 27 % Isopropanol, pH 6,9 ± 0,2 |
| Verdünnungspuffer | 20 mM Tris-HCl, 5 mM CaCl₂, pH 6,9 ± 0,2 |

### 4. Hydroxyapatit Ultrogel-Chromatographie

### 4.1 Trennprinzip

Hydroxyapatit Ultrogel (Biosepra) besteht aus Hydroxyapatit (kristallines Kalziumphosphat), das in einem Agarosegerüst eingelagert ist, um seine mechanischen und hydrodynamischen Eigenschaften zu verbessern. EPO bindet an diese Matrix und wird bei einer niedrigeren Phosphatkonzentration als die meisten Proteinverunreinigungen eluiert.

### 4.2 Durchführung

Die Chromatographiesäule (Amicon P440 x 500 oder Äquivalent) wird mit 30 - 40 1 Hydroxyapatit Ultrogel gepackt und mit 0,5 N NaOH regeneriert. Anschließend wird die Säule mit mindestens 4 SV Äquilibrierpuffer äquilibriert.

Das Eluat der Butyl-Toyopearl Säule wird bei einer Temperatur von ca. 15°C und einer Flußrate von 500 - 1200 ml/min auf die Säule aufgezogen. Die Säule wird bei gleicher Temperatur und Flußrate mit ca. 1 SV Äquilibrierpuffer und dann mit ca. 2 SV Waschpuffer nachgewaschen. Anschließend wird sie mit ca. 3 SV Elutionspuffer eluiert. Der gesamte Proteinpeak wird gesammelt (ca. 10 - 18 l) und bis zur Weiterverarbeitung bei 15°C gelagert. Bei dieser Chromatographie wird eine Reinheit von besser 95 % erreicht.

| | |
|---|---|
| Äquilibrierpuffer: | 20 mM Tris-HCl, 5 mM CaCl₂, 0,25 M NaCl, 9 % Isopropanol, pH 6,9 ± 0,2 |
| Waschpuffer: | 10 mM Tris-HCl, 5 mM CaCl₂, pH 6,8 ± 0,2 |
| Elutionspuffer: | 10 mM Tris-HCl, 10 mM K-Phosphat, 0,5 mM CaCl₂, pH 6,8 ± 0,2 |

### 5. Reversed Phase HPLC (RP-HPLC)

### 5.1 Trennprinzip

Das RP-HPLC-Material z.B. Vydac C4 (Vydac, USA) besteht aus Kieselgelpartikeln, deren Oberfläche C4-Alkylketten trägt. Aufgrund hydrophober Wechselwirkungen bindet EPO an diese Matrix und wird mit einem Acetonitrilgradienten in verdünnter Trifluoressigsäure selektiv eluiert.

### 5.2 Durchführung

Die präparative HPLC wird mit einer Merck Prepbar 100 -Trennanlage (oder Äquivalent) bei einer Temperatur von 22 ± 4 °C durchgeführt. Die Trennsäule (100 mm x 400 mm, 3,2 l) ist mit Vydac C4-Material gepackt. Vor dem Einsatz wird die Säule durch mehrfaches Anlegen eines Gradienten von Puffer A nach 100 % Lösungsmittel regeneriert und anschließend mit Puffer A äquilibriert.

Das Eluat der Hydroxyapatit-Säule wird mit Trifluoressigsäure auf ca. pH 2,5 angesäuert und sterilfiltriert. Anschließend wird es bei einer Temperatur von 22 ± 4 °C und einer Flußrate von 250 - 310 ml/min auf die Säule aufgezogen. Die Säule wird bei gleicher Temperatur und Flußrate mit einem linearen Gradient von Puffer A nach Puffer B eluiert. Der Elutionspeak wird in Fraktionen aufgefangen. Durch Vorlegen von 4 Volumina HPLC-Verdünnungspuffer wird das Eluat sofort neutralisiert.

Fraktionen, die bei der analytischen HPLC eine Reinheit von mindestens 99 % aufweisen, werden vereinigt (Poolvolumen ca. 4 - 6 1). Bei dieser Chromatographie werden Spurenverunreinigungen abgetrennt und eine Reinheit von besser 99 % erreicht.

| | |
|---|---|
| Puffer A | 0,1 % Trifluoressigsäure in Wasser |
| Puffer B | 80 % Acetonitril, 0,1 % Trifluoressigsäure in Wasser |
| HPLC-Verdünnungspuffer | 10 mM Na/K-Phosphat, pH 7,5 ± 0,2 |

### 6. DEAE-Sepharose ff Chromatographie

### 6.1 Trennprinzip

DEAE Sepharose fast flow (Pharmacia) besteht aus DEAE-Gruppen, die kovalent an die Oberfläche von Sepharosekügelchen gebunden sind. Aufgrund ionischer Wechselwirkungen bindet EPO an diese Matrix und wird durch Erhöhung der Ionenstärke eluiert.

### 6.2 Durchführung

Die Chromatographiesäule (Amicon P90 x 250 oder Äquivalent) wird mit 100 - 200 ml Gel pro g EPO im Auftrag gefüllt und mit 0,5 N NaOH regeneriert. Anschließend wird die Säule zunächst mit 100 mM Na/K-Phosphatpuffer, pH 7,5 und dann mit mindestens 12 SV Äquilibrierpuffer äquilibriert.

Das Eluat der HPLC-Säule wird bei einer Temperatur von 5 ± 4 °C und einer Flußrate von ca. 150 ml/min auf die Säule aufgezogen. Die Säule wird bei gleicher Temperatur und Flußrate mit mindestens 5 SV Äquilibrierpuffer und dann mit ca. 10 SV Waschpuffer gewaschen. Anschließend wird sie erneut mit ca. 10 SV Äquilibrierpuffer gewaschen und dann mit ca. 7 SV Elutionspuffer eluiert. Der gesamte Proteinpeak wird gesammelt (ca. 2-51), sterilfiltriert und abgefüllt.

Bei dieser Chromatographie wird das Lösungsmittel aus dem HPLC-Schritt abgetrennt und Spurenverunreinigungen entfernt. Die Reinheit ist besser 99 %.

| | |
|---|---|
| Äquilibrierpuffer: | 10 mM Na/K-Phosphat, pH 7,5 ± 0,2 |
| Waschpuffer: | 30 mM Na-Acetat, pH 4,5 ± 0,1 |
| Elutionspuffer: | 10 mM Na/K-Phosphat, 80 mM NaCl pH 7,5 ± 0,2 |

### Referenzliste

Bavister, B., J. Expcology 217 (1981) 45-51
EP-A 0 248 656
EP-A 0 267 678
EP-A 0 307 247
EP-A 0 343 635
EP-A 0 481 791
EP-A 0 513 738
EP-B 0 148 605
EP-B 0 205 564
EP-B 0 209 539
EP-B 0 411 678
Huang, S.L., PNAS, USA 81 (1984) 2708-2712
Kawamoto, T. et al., Analytical Biochem. 130 (1983) 445-453
Kowar, J. und Franek, F., Methods in Enzymology 421 (1986) 277-292
Lai, P.H. et al., J. Biol. Chem. 261 (1986) 3116-3121
Nobuo, I., et al., J. Biochem. 107 (1990) 352-359
Protein Purification Methods, A practical approach, Ed. Harris, E.L.V., and Angal S., IRL Press, Oxford, England (1989) Seite 224
Protein Purification, Ed. Janson, J.C., Ryden, L., VCH-Verlag, Weinheim, Deutschland (1989), Seiten 207-226.
Sasaki, H. et al., J. Biol. Chem 262 (1987) 12059-12076
WO 86/07494
WO 88/00967
WO 91/09955
WO 93/09222

## Patentansprüche

1. Verfahren zur Herstellung eines Proteinpräparats mit Erythropoietinaktivität, welches **gekennzeichnet ist durch**
a) einen Gehalt von Proteinen, die aus der Wirtszelle stammen und keine biologische Funktion von Erythropoietin besitzen, von ≤ 100 ppm
b) einen Gehalt von DNA aus der Wirtszelle von ≤ 10 pg/83 µg Erythropoietin und **dadurch**, daß
c) das Präparat vollständig frei von natürlichen Säugerproteinen ist, die nicht aus der Wirtszelle stammen,
**durch** Expression einer für das Protein codierenden DNA in einer eukaryontischen Wirtszelle, Kultivierung der Wirtszelle in einem Medium, welches frei von natürlichen Säugerproteinen ist, und chromatographischer Reinigung des Proteins aus dem Zellüberstand, **dadurch gekennzeichnet**, daß die chromatographische Reinigung im ersten Schritt als Farbstoffaffinitätschromatographie, im zweiten Schritt als hydrophobe Chromatographie, im weiteren Schritt als Chromatographie an Hydroxyapatit, im weiteren Schritt als reversed phase Chromatographie an einem hydrophoben Träger und als nächstem Schritt als Anionenaustauschchromatographie erfolgt.

2. Verfahren nach Anspruch 1, **dadurch gekennzeichnet, daß** die hydrophobe Chromatographie an einem butylierten Träger erfolgt.

3. Verfahren nach den Ansprüchen 1 bis 2, **dadurch gekennzeichnet, daß** die reversed phase Chromatographie an einem butylierten Träger bei neutralem pH-Wert erfolgt.

4. Verfahren nach den Ansprüchen 1 bis 3, **dadurch gekennzeichnet, daß** die Beladung und/oder Elution der hydrophoben Chromatographie in Gegenwart eines Alkohols erfolgt.

5. Verfahren nach Anspruch 4, **dadurch gekennzeichnet, daß** zur Elution Isopropanol in einer Menge von 25 - 35 % (v/v) verwendet wird.

6. Verfahren nach Anspruch 4, **dadurch gekennzeichnet, daß** zur Beladung Isopropanol in einer Menge von 10 - 15 % (v/v) verwendet wird.

## Claims

1. Process for producing a protein preparation having erythropoietin activity, which is **characterized by**
a) a content of proteins which are derived from the host cell and have no biological function of erythropoietin of ≤ 100 ppm
b) a content of DNA from the host cell of ≤ 10 pg/83 µg erythropoietin and **characterized in that**
c) the preparation is completely free of natural mammalian proteins which are not derived from the host cell,
by expressing a DNA coding for the protein in a eukaryotic host cell, culturing the host cell in a medium which is free of natural mammalian proteins and chromatographically purifying the protein from the cell supernatant, **characterized in that** the chromatographic purification is carried out in a first step as dye affinity chromatography, in a second step as hydrophobic chromatography, in a further step as a chromatography on hydroxyapatite, in a further step as reversed phase chromatography on a hydrophobic support and as the next step as anion exchange chromatography.

2. Process as claimed in claim 1, **characterized in that** the hydrophobic chromatography is carried out on a butylated support.

3. Process as claimed in claims 1 to 2, **characterized in that** the reversed phase chromatography is carried out on a butylated support at a neutral pH.

4. Process as claimed in claims 1 to 3, **characterized in that** the loading and/or elution of the hydrophobic chromatography is carried out in the presence of an alcohol.

5. Process as claimed in claim 4, **characterized in that** isopropanol is used for the elution in an amount of 25 - 35 % (v/v).

6. Process as claimed in claim 4, **characterized in that** isopropanol is used for the loading in an amount of 10 - 15 % (v/v).

## Revendications

1. Procédé de préparation de protéine qui a l'activité d'érythropoïétine, **caractérisé en ce que**
a) une teneur en protéines, obtenue à partir de la cellule hôte et qui ne possèdent pas de fonction biologique d'érythropoïétine, qui est ≤ 100 ppm
b) une teneur d'ADN de la cellule hôte par rapport à d'érythropoïétine, qui est ≤ 10 pg/83 µg
et **caractérisé en ce que**
c) la préparation ne contient pas de protéines naturelles animales qui ne proviennent pas de la cellule hôte
**caractérisé en ce qu'**il utilise une expression d'ADN codant pour la protéine dans une cellule d'eucaryote hôte, une production de cellules hôte sans protéines naturelles animales, et la chromatographie pour une purification des protéines de la cellule ; la chromatographie d'affinité de colorant est utilisée pour la purification, la chromatographie hydrophobe dans la deuxième étape de la purification, la chromatographie avec une hydroxyapatite dans une autre étape, la chromatographie de phase inversée avec un remplissage hydrophobe dans une autre étape, et la chromatographie d'échange d'anions peut être utilisée dans une étape ultérieure.

2. Procédé selon la revendication 1, **caractérisé en ce que** la chromatographie hydrophobe est effectuée en utilisant un remplissage butylique.

3. Procédé selon les revendications 1 à 2, **caractérisé en ce que** la chromatographie de phase inversée utilise un remplissage butylique et est effectuée à un pH physiologique neutre.

4. Procédé selon les revendications 1 à 3, **caractérisé en ce que** une injection et/ou une élution dans la chromatographie hydrophobe sont effectuées en présence d'alcool.

5. Procédé selon la revendication 4, **caractérisé en ce que** l'isopropanol est utilisé dans l'élution en quantité de 25-35% en volume.

6. Procédé selon la revendication 4, **caractérisé en ce que** l'isopropanol est utilisé dans l'injection en quantité de 10-15% en volume.
